# EUROPÄISCHE PATENTSCHRIFT

(11) **EP 1 329 231 B1**
(45) Veröffentlichungstag und Bekanntmachung des Hinweises auf die Patenterteilung: **20.08.2003**
(21) Anmeldenummer: 02001463.5
(22) Anmeldetag: 21.01.2002
(51) Int. Cl.: A61M 1/00, A61B 19/00, A61B 1/015, A61M 39/22, F16K 1/14

(54) **Medizinisches Instrument zum Spülen und/oder Saugen**
Medical instrument for irrigation and/or suction
Instrument médical pour l'irrigation et/ou l'aspiration

(43) Veröffentlichungstag der Anmeldung: 23.07.2003
(73) Patentinhaber: Karl Storz GmbH & Co. KG, 78532 Tuttlingen (DE)
(72) Erfinder: Bacher, Uwe, 78532 Tuttlingen (DE); Dittrich, Horst, 78194 Immendingen (DE)
(74) Vertreter: Heuckeroth, Volker, Dr.

(56) Entgegenhaltungen:
- FR-A- 2 646 771
- GB-A- 1 346 057
- US-A- 1 334 870
- US-A- 5 030 199
- US-A- 5 312 373

## Beschreibung

Die Erfindung betrifft ein medizinisches Instrument zum Spülen und/oder Saugen, mit einem Instrumentenkörper, in dem zumindest ein Flußkanal für den Durchtritt eines Fluids vorhanden ist, und mit zumindest einem Absperrorgan zum wahlweisen Absperren des zumindest einen Flußkanals, und mit einem Betätigungsorgan zum Betätigen des Absperrorgans, wobei das zumindest eine Absperrorgan eine Kugel ist, die in Schließstellung an einen Dichtrand des Flußkanals dichtend anliegt, so daß sie den Innenquerschnitt des Flußkanals verschließt, und wobei das Betätigungsorgan einen Druckknopf zur Bedienung des Betätigungsorgans aufweist.

Ein derartiges Instrument ist aus dem Patent US 5 312 373 A bekannt.

Ein Saug- und/oder Spülinstrument der eingangs genannten Art wird in der Chirurgie, insbesondere in der minimal-invasiven Chirurgie, dazu verwendet, bei einem chirurgischen Eingriff das Operationsgebiet mit Spülflüssigkeit zu spülen, und/oder um Flüssigkeit, beispielsweise die eingeführte Spülflüssigkeit, Blut und Gewebereste aus dem Operationsgebiet abzusaugen.

Das zuvor genannte bekannte Instrument eignet sich sowohl zum Spülen als auch zum Absaugen. Während die vorliegende Erfindung bevorzugt am Beispiel eines Instrumentes zum Spülen und Absaugen beschrieben wird, ist die vorliegende Erfindung jedoch nicht hierauf beschränkt, sondern umfaßt auch solche Instrumente, mit denen entweder nur gespült oder nur gesaugt werden kann.

Ebenso ist der Begriff "Instrumentenkörper" im Sinne der vorliegenden Erfindung allgemein zu verstehen und kann den Schaft eines solchen Instruments und/oder den Handgriff eines solchen Instruments umfassen.

Das bekannte Instrument ist eine endoskopische Kanülenanordnung, mit der eine Flüssigkeit oder Gas in einen Körperhohlraum während eines chirurgischen Eingriffs eingeführt oder von diesem abgeführt werden kann.

Am proximalen Ende des Instruments weist das bekannte Instrument einen Anschluß zum Anschließen eines Saugschlauches und einen Anschluß zum Anschließen eines Spülschlauches auf. Der Saugschlauch und der Spülschlauch werden mit einer Saug- und Spülquelle verbunden, wobei an dem Saugschlauch ein entsprechender Unterdruck angelegt wird, der vom distalen Ende zum proximalen Ende des Instruments gerichtet ist, und wobei durch den Spülschlauch Spülflüssigkeit von proximal nach distal geleitet wird. Das bekannte Instrument weist einen Ventilmechanismus auf, der ein Betätigungsorgan aufweist, das als Bedienungselement einen Druckknopf aufweist, der mit dem Finger oder Daumen bedient werden kann. Als Absperrorgan weist dieser Ventilmechanismus eine Kugel auf, die in dem Flußkanal angeordnet ist. Die Anordnung ist dabei so getroffen, daß durch Herunterdrücken des Druckknopfes die Kugel in Flußrichtung des Flußkanals bewegt wird, um das gewünschte Spülen oder Saugen zu ermöglichen.

Aus dem Patent US 1 334 870 A ist weiterhin ein Ventil bekannt, das als Absperrorgan eine Kugel aufweist, die zum Freigeben eines Flußkanals in Richtung quer zur Längsrichtung des Flußkanals bewegbar ist. Der Drehknopf wirkt über ein Gewinde mit einem Kopfstück des Ventils zusammen, so daß durch Drehen des Drehknopfs eine Aufnahme für die Kugel aus der Schließstellung in die Freigabestellung und umgekehrt bewegt werden kann. Diese Ventilanordnung ist nicht zur Verwendung in medizinischen Instrumenten vorgesehen.

Eine Ventilanordnung zur Verwendung in einem Sichtmeßgerät für Flüssigkeitstanks, beispielsweise Öltanks, ist in der GB 1 346 057 A beschrieben. Diese Ventilanordnung weist als Absperrorgan ebenfalls eine Kugel auf, die über eine O-Ring-Dichtung an einem Dichtrand eines Flußkanals anliegt. Die Kugel ist quer zur Längsrichtung des Flußkanals mittels eines Druckknopfes betätigbar.

Aus dem Patent US 5 030 199 A ist ein medizinisches Instrument zur Kontrolle von Inkontinenz beschrieben, die ein magnetisch betätigbares Steuerventil aufweist, das als Absperrorgan eine Kugel aufweist, die aus Silikon besteht.

Ferner ist aus dem deutschen Gebrauchsmuster DE 200 11 409 U1 ein Saug- und Spülinstrument bekannt. Um bei diesem bekannten Instrument den Saugstrom und/oder den Spülstrom unterbrechen zu können, ist ein Absperrorgan zum wahlweisen Absperren bzw. Freigeben des jeweiligen Flußkanals vorgesehen, wobei das Absperrorgan über ein Betätigungsorgan in Form eines Schiebers von der Bedienungsperson betätigt werden kann. Das Absperrorgan weist eine mit dem Schieber verbundene, mit Aussparungen versehene bewegliche Platte auf, die auf einer unbeweglichen Platte im Instrumentenkörper gleitend angeordnet ist. In der unbeweglichen Platte sind mit dem zumindest einen Flußkanal kommunizierende Öffnungen ausgebildet. Durch Betätigen des Schiebers wird die bewegliche Platte relativ zur unbeweglichen Platte verschoben, so daß die Aussparungen der beweglichen Platte und die Öffnungen der unbeweglichen Platte in oder außer Deckung kommen. Auf diese Weise kann der Saugstrom bzw. der Spülstrom freigegeben werden oder es kann der Saugkanal und/oder der Spülkanal abgesperrt werden. Die bewegliche Platte und die unbewegliche Platte liegen, sich gegenseitig flächig berührend, unmittelbar aufeinander, wobei beide Platten im Bereich ihrer sich gegenseitig berührenden Oberflächen aus einem keramischen Material gebildet sind.

Nachteilig an dieser bekannten Konstruktion ist, daß derartige keramische Scheiben in der Gestehung sehr teuer sind. Des weiteren benötigen derartige flächig aufeinanderliegende keramische Scheiben bei hohen Saug- oder Spüldrücken hohe Zuhaltekräfte, um die erforderliche Dichtwirkung zu erzielen. Derartige hohe Zuhaltekräfte erzeugen wiederum eine hohe Reibung zwischen der beweglichen und der unbeweglichen Platte, woraus hohe Bedienkräfte bei der Betätigung des Betätigungsorgans resultieren.

Der Erfindung liegt die Aufgabe zugrunde, ein Instrument der eingangs genannten Art dahingehend weiterzubilden, daß eine hohe Dichtwirkung des Absperrorgans erzielt wird und daß das Instrument auf leicht handhabbare Weise gründlich reinigbar ist.

Erfindungsgemäß wird diese Aufgabe hinsichtlich des eingangs genannten Instruments dadurch gelöst, daß die Kugel formelastisch ist, daß die Kugel zwischen der Schließstellung zum Verschließen des Flußkanals und einer Freigabestellung zum Freigeben des Flußkanals im wesentlichen in Richtung quer zur Längsrichtung des Flußkanals bewegbar ist, und daß das Betätigungsorgan zum Bewegen der Kugel zwischen der Schließstellung und der Freigabestellung ebenfalls im wesentlichen quer zur Längsrichtung des Flußkanals bewegbar ist, daß das Betätigungsorgan zusammen mit der Kugel vom Instrumentenkörper abnehmbar ist.

Das erfindungsgemäße Instrument weist demnach eine Art Kugelventil auf. Die Kugel weist dabei einen Außendurchmesser auf, der größer ist als der Innendurchmesser des zumindest einen Flußkanals, den die Kugel in der Schließstellung verschließen soll. Die Kugel liegt somit kreislinienförmig bzw. ringsektorförmig an dem Dichtrand des Flußkanals dichtend an, wodurch im Unterschied zu den flächig aufeinanderliegenden keramischen Platten bereits mit einem geringen Anlagedruck der Kugel an dem Dichtrand des Flußkanals eine hohe Dichtwirkung erzielt wird. Um den zumindest einen Flußkanal freizugeben, ist erfindungsgemäß vorgesehen, daß die Kugel im wesentlichen in Richtung quer zur Längsrichtung des Flußkanals in eine Freigabestellung bewegbar ist, wobei diese Bewegung durch Bedienung des Betätigungsorgans ebenfalls in Richtung quer zum Flußkanal bewirkt wird, was im Unterschied zu den keramischen Platten des bekannten Instruments geringere Bedienkräfte erfordert, weil im wesentlichen kein Reibschluß bei der Bewegung der Kugel zu überwinden ist.

Die formelastische Ausgestaltung der Kugel trägt vorteilhafterweise zu einer verbesserten Dichtwirkung gegenüber dem Dichtrand des Flußkanals bei, weil sich die Kugel aufgrund der Formelastizität innig an den Dichtrand des Flußkanals anschmiegen kann und Oberflächenunregelmäßigkeiten oder -rauhigkeiten der Kugel oder des Dichtrands des Flußkanals sich nicht nachteilig auf die Dichtwirkung des Absperrorgans auswirken können.

Die Abnehmbarkeit des Betätigungsorgans zusammen mit der Kugel hat zum einen den Vorteil, daß die Kugel im Falle eines Verschleißes ausgewechselt werden kann, und zum anderen, daß das Absperrorgan und das Betätigungsorgan nach Entnahme von dem Instrumentenkörper leicht gereinigt werden können. Insbesondere im Fall, daß der zumindest eine Flußkanal ein Saugkanal zum Absaugen von Flüssigkeiten, Geweberesten und dergleichen ist, können sich im Bereich des Absperrorgans Verunreinigungen ansammeln, die aufgrund der Abnehmbarkeit des Betätigungsorgans und der Kugel wesentlich leichter entfernt werden können. Auf diese Weise erfüllt das erfindungsgemäße Instrument auch die an medizinische Instrumente im allgemeinen gestellten hohen Anforderungen an die Desinfizier- bzw. Reinigbarkeit solcher Instrumente.

In einer bevorzugten Ausgestaltung der Erfindung ist die Kugel stromaufwärts des Dichtrands des Flußkanals angeordnet.

Diese Maßnahme hat den Vorteil, daß im Falle, daß der zumindest eine Flußkanal ein Saugkanal ist, die Kugel allein aufgrund des Saugdruckes bzw. im Fall, daß der zumindest eine Flußkanal ein Spülkanal ist, die Kugel allein durch den Spüldruck gegen den Dichtrand des Flußkanals gedrückt werden kann. Konstruktiv aufwendige Mittel zum Andrücken der Kugel an den Dichtrand des Flußkanals, beispielsweise Federn oder dergleichen, sind bei dieser Bauweise vorteilhafterweise nicht erforderlich. Des weiteren wird durch diese Maßnahme erreicht, daß die Anpressung der Kugel an den Dichtrand mit zunehmendem Saug- oder Spüldruck sogar erhöht wird, wodurch sich die zum sicheren Absperren des Flußkanals erforderliche Zuhaltekraft von selbst einstellt.

In einer weiteren bevorzugten Ausgestaltung ist die Kugel in einer Aufnahme des Betätigungsorgans angeordnet, in der die Kugel eine Beweglichkeit in Längsrichtung des Flußkanals aufweist.

Hierbei ist von Vorteil, daß die Kugel bei ihrer Bewegung von der Schließstellung in die Freigabestellung und umgekehrt aufgrund ihrer axialen Beweglichkeit leichter am Dichtrand des Flußkanals vorbeibewegt werden kann. Diese Maßnahme trägt somit vorteilhafterweise zu einer Bedienung des Betätigungsorgans mit weiter verminderter Bedienkraft bei.

In einer weiteren bevorzugten Ausgestaltung ist die Kugel um im wesentlichen ihren Mittelpunkt drehbar in der Aufnahme gelagert.

Auch diese Maßnahme trägt vorteilhafterweise zu einer Verringerung der Bedienkraft des Betätigungsorgans bei, weil die Kugel aufgrund dieser Ausgestaltung bei ihrer Bewegung von der Schließstellung in die Freigabestellung und umgekehrt an dem Dichtrand des Flußkanals abrollen kann.

In einer weiteren bevorzugten Ausgestaltung ist die Beweglichkeit der Kugel in der Aufnahme in Längsrichtung des Flußkanals entgegen der Flußrichtung des Fluids begrenzt.

Während die Beweglichkeit der Kugel in Längsrichtung des Flußkanals in der Flußrichtung des Fluids durch den Dichtrand des Flußkanals begrenzt ist, hat diese Maßnahme den Vorteil, daß die Kugel in einem Zustand des Instruments, in dem kein Fluiddruck, beispielsweise Saug- oder Spüldruck ansteht, sich im Instrumentenkörper nicht vom Betätigungsorgan entfernen kann.

In einer weiteren bevorzugten Ausgestaltung ist die Kugel in Richtung quer zum Flußkanal im wesentlichen unbeweglich in der Aufnahme gehalten.

Hierbei ist von Vorteil, daß die Kugel zumindest in der Schließstellung stets die für die Erzielung einer hohen Dichtwirkung günstige zentrierte Position bezüglich des Dichtrand des Flußkanals einnimmt.

In einer weiteren bevorzugten Ausgestaltung ist das Betätigungsorgan in die Schließstellung oder die Freigabestellung der Kugel vorgespannt.

Eine derartige Vorspannung des Betätigungsorgans hat den Vorteil, daß zumindest eine der beiden Betriebsstellungen der Kugel, d.h. die Schließstellung oder die Freigabestellung selbsttätig durch Loslassen eingenommen wird, was die Bedienungsfreundlichkeit des erfindungsgemäßen Instruments verbessert. Vorzugsweise kann die Vorspannung mittels einer am Betätigungsorgan vorgesehenen Feder bewerkstelligt sein.

In einer weiteren bevorzugten Ausgestaltung ist vorgesehen, daß am Betätigungsorgan und am Instrumentenkörper ein Verriegelungsmechanismus zum Verriegeln des Betätigungsorgans am Instrumentenkörper in Form eines Bajonettverschlusses vorhanden ist.

Hierdurch läßt sich das Abnehmen des Betätigungsorgans und der Kugel in leicht handhabbarer Weise durchführen, wodurch das Instrument besonders schnell zerlegt und wieder zusammengesetzt werden kann, so daß das Instrument besonders leicht und schnell einer Reinigung zugänglich ist.

In einer weiteren bevorzugten Ausgestaltung ist zum Verriegeln und zum Entriegeln des Verriegelungsmechanismus ein um eine Längsachse des Betätigungsorgans verschwenkbarer Hebel vorgesehen.

Diese Maßnahme führt zu einer noch leichter zu handhabenden und schnelleren Entnahme bzw. Montage des Betätigungsorgans und der Kugel.

In einer weiteren bevorzugten Ausgestaltung sind in dem Instrumentenkörper zumindest zwei Flußkanäle vorhanden, wobei jedem Flußkanal ein Absperrorgan und ein Betätigungsorgan zugeordnet ist.

Mit dieser Ausgestaltung kann ein kombiniertes Saug- und Spülinstrument realisiert werden, wobei durch eine leicht durchführbare Betätigung des jeweiligen Betätigungsorgans zwischen den Funktionen Spülen, Saugen bzw. Absperren sowohl des Saug- als auch des Spülkanals umgeschaltet werden kann.

In einer weiteren bevorzugten Ausgestaltung ist alternativ zu der oben erwähnten frei drehbaren Lagerung der Kugel in der Aufnahme des Betätigungsorgans vorgesehen, daß die Kugel in Richtung zum Dichtrand des Flußkanals hin vorgespannt ist.

Diese Maßnahme ist insbesondere vorteilhaft, wenn in dem Flußkanal ein geringer Druck bzw. ein geringer Druckgradient herrscht, da durch die Vorspannung die Kugel an den Dichtrand gedrückt wird, wodurch eine ausreichende Dichtwirkung der Kugel an dem Dichtrand des Flußkanals erzielt wird.

Weitere Vorteile und Merkmale ergeben sich aus der nachfolgenden Beschreibung und beigefügten Zeichnung.

Es versteht sich, daß die vorstehend genannten und nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden mit Bezug auf diese hiernach näher beschrieben. Es zeigen:
- Fig. 1: eine perspektivische Gesamtansicht eines medizinischen Instruments zum Spülen und Saugen, wobei ein Schaft des Instruments nur teilweise dargestellt ist;
- Fig. 2: einen Schnitt durch das Instrument in Fig. 1 entlang der Linie II-II in Fig. 1, wobei ein Absperrorgan des Instruments in seiner Schließstellung dargestellt ist;
- Fig. 3: eine Fig. 2 entsprechende Darstellung des Instruments, wobei das Absperrorgan in seiner Freigabestellung dargestellt ist;
- Fig. 4: eine mit Fig. 1 vergleichbare Darstellung des Instruments mit abgenommenen Absperr- und Betätigungsorganen in explosionsartiger Darstellung; und
- Fig. 5: eine äußerst schematische Teildarstellung des Betätigungsorgans und der Kugel in einer geringfügig abgewandelten Ausführungsform.

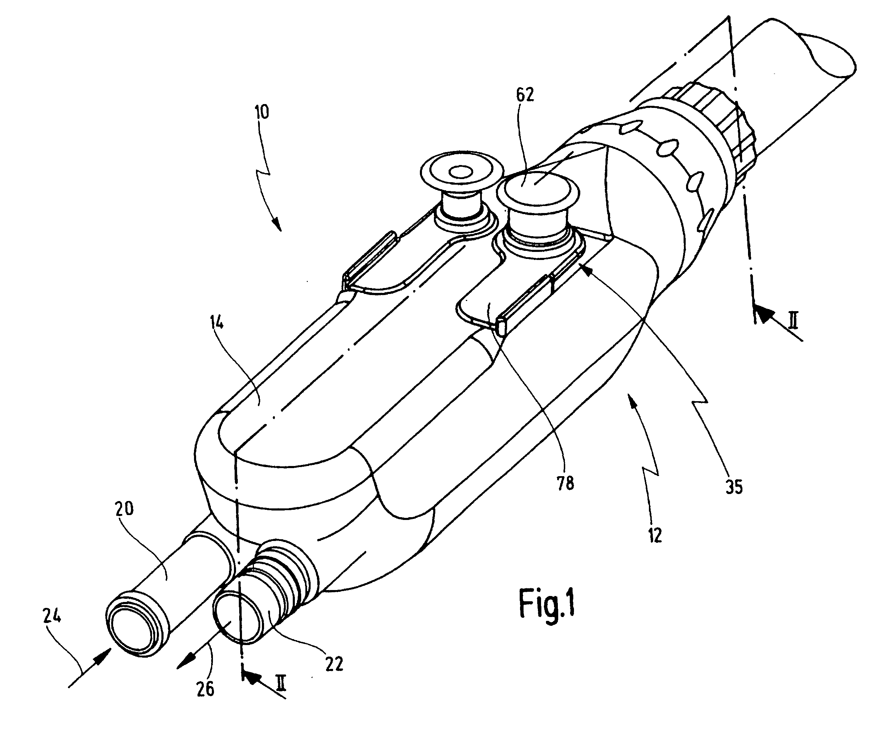

In Figuren 1 bis 4 ist ein mit dem allgemeinen Bezugszeichen 10 versehenes medizinisches Instrument dargestellt, das in dem gezeigten Ausführungsbeispiel sowohl zum Spülen als auch zum Saugen verwendet werden kann.

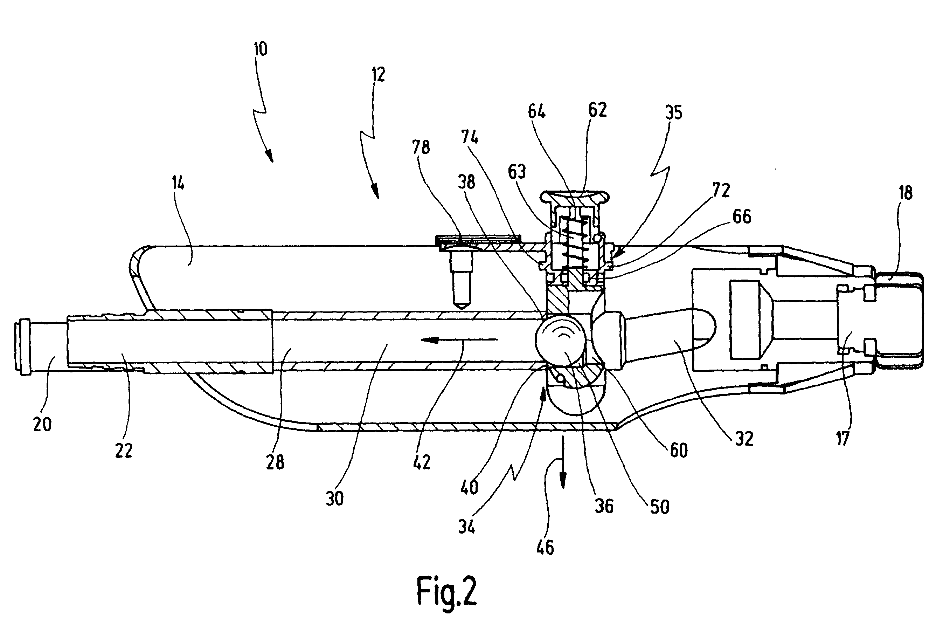

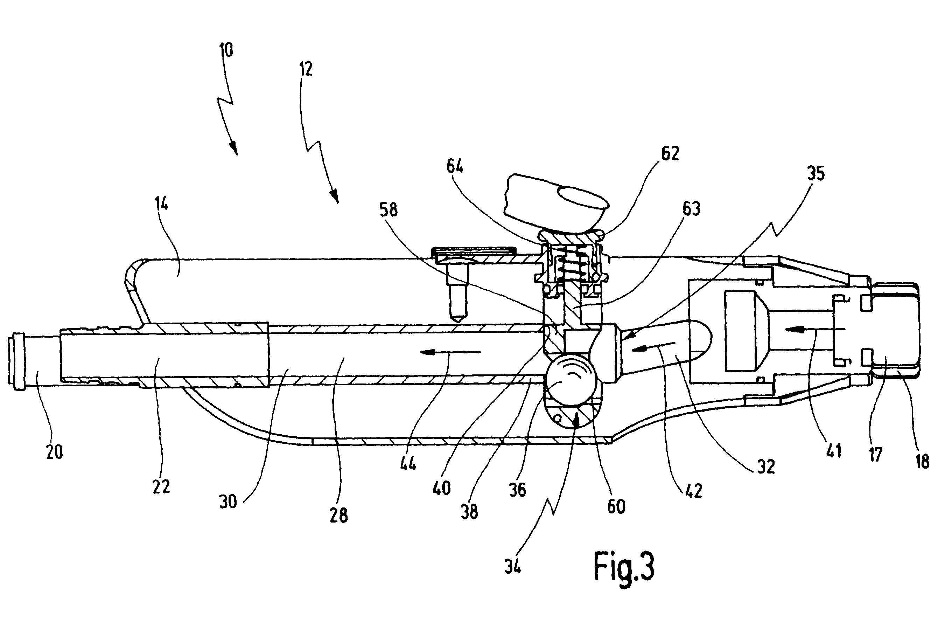

Das Instrument 10 weist allgemein einen Instrumentenkörper 12 auf, der einen Handgriff 14 und einen nur in Fig. 1 angedeuteten Schaft 16 aufweist. Der Handgriff 14 ist im wesentlichen stabförmig ausgebildet. Der Schaft 16 kann mit dem Handgriff 14 über eine Aufnahme 17 (Fig. 2 und 3) am distalen Ende des Handgriffs 14 verbunden und mittels einer Fixierschraube 18 gesichert werden.

Am proximalen Ende des Handgriffs 14 sind ein Spülanschluß 20 und ein Sauganschluß 22 in Draufsicht nebeneinanderliegend angeordnet.

Mittels eines nicht dargestellten Spülschlauchs, der an den Spülanschluß 20 angeschlossen wird, und mittels eines nicht dargestellten Saugschlauchs, der an den Sauganschluß 22 angeschlossen wird, kann das Instrument 10 mit einer nicht dargestellten Saug- und Spülquelle verbunden werden. Gemäß einem Pfeil 24 in Fig. 1 wird ein Fluid, beispielsweise Spülflüssigkeit, von der Spülquelle kommend dem Spülanschluß 20 zugeführt, und gemäß einem Pfeil 26 in Fig. 1 wird an den Sauganschluß 22 ein Unterdruck an das Instrument 10 angelegt.

Von dem Spülanschluß 20 und von dem Sauganschluß 22 ausgehend, erstreckt sich durch das Instrument 10 jeweils ein Flußkanal für den Durchtritt eines Fluids, d.h. ausgehend von dem Spülanschluß 20 erstreckt sich ein in den Figuren nicht näher dargestellter, als Spülkanal dienender Flußkanal durch den Instrumentenkörper 12, und von dem Sauganschluß 22 ausgehend erstreckt sich ein als Saugkanal dienender Flußkanal 28 durch den Instrumentenkörper 12 hindurch. Der Längsschnitt gemäß Figuren 2 und 3 ist durch den Flußkanal 28 hindurch gelegt, während der in Figuren 2 und 3 nicht dargestellte Spülkanal von dem Flußkanal 28 getrennt und parallel zu diesem verläuft.

Der Flußkanal 28 (das gleiche gilt für den weiteren Flußkanal zum Spülen) erstreckt sich durch den Handgriff 14 hindurch zu dem Schaft 16 und verläuft weiterhin durch den Schaft 16 hindurch bis zu dessen nicht dargestelltem distalen Ende. In dem Schaft 16 können die Flußkanäle weiterhin voneinander getrennt verlaufen, können jedoch auch in dem Schaft 16 zusammengeführt sein.

Die folgende Beschreibung beschränkt sich auf die Beschreibung des Flußkanals 28 (Saugkanal) und die mit diesem zusammenwirkenden Elemente des Instruments 10. Der nicht näher dargestellte weitere Flußkanal (Spülkanal) und die mit diesem zusammenwirkenden Elemente unterscheiden sich von dem Flußkanal 28 und den mit diesem zusammenwirkenden Elementen konstruktiv nicht, soweit in der folgenden Beschreibung nichts anderes angegeben ist.

In den Flußkanal 28 ist ein Absperrorgan 34 zum wahlweisen Absperren und Freigeben des Flußkanals 28 geschaltet, das nachfolgend noch näher beschrieben wird. Das Absperrorgan 34 ist dabei im Handgriff 14 des Instruments 10 angeordnet und unterteilt den Flußkanal 28 zum Zwecke der einfacheren Beschreibung in einen proximalen Abschnitt 30 und einen distalen Abschnitt 32.

Das Absperrorgan 34 ist über ein Betätigungsorgan 35, das ebenfalls nachfolgend noch näher beschrieben wird, betätigbar, wobei das Absperrorgan 34 zwischen einer Schließstellung (Fig. 2) und einer Freigabestellung (Fig. 3) bewegbar ist.

Das Absperrorgan 34 ist als Kugel 36 ausgebildet und einem distalen Ende 38 des proximalen Abschnitts 30 des Flußkanals 28 benachbart angeordnet.

Der Flußkanal 28 weist am distalen Ende 38 seines proximalen Abschnitts 30 einen Dichtrand 40 auf, der ein stirnseitiger Rand des proximalen Abschnitts 30 ist, und an dem die Kugel 36 in ihrer Schließstellung gemäß Fig. 2 dichtend anliegt, so daß die Kugel 36 den Innenquerschnitt des Flußkanals 28 verschließt. Dazu ist zumindest das distale Ende 38 bzw. der Dichtrand 40 im Querschnitt rund ausgebildet, so daß die Kugel 36 über eine geschlossene Kreislinie bzw. einen geschlossenen Kreisring an dem Dichtrand 40 des Flußkanals 28 dichtend anliegt. Die Kugel 36 weist entsprechend einen Durchmesser auf, der größer ist als der Innendurchmesser des Flußkanals 28.

Die Kugel 36 wird in der Schließstellung gemäß Fig. 2 allein aufgrund des in dem Flußkanal 28 herrschenden Saug- bzw. Strömungsdrucks gemäß einem Pfeil 42 gegen den Dichtrand 40 des Flußkanals 28 gedrückt. Dies wird dadurch erreicht, daß die Kugel 36 bzgl. der durch den Flußkanal 28 hindurchgehenden Strömungsrichtung oder Richtung des Druckgefälles des Fluids stromaufwärts des Dichtrands 40 des Flußkanals 28 angeordnet ist.

In der in Fig. 2 dargestellten Schließstellung der Kugel 36 kann somit kein Fluid durch den Flußkanal 28 hindurchtreten, d.h. der Saug- bzw. Fluidstrom durch den Flußkanal 28 ist in dieser Schließstellung unterbrochen. Zum Freigeben des Flußkanals 28, im vorliegenden Fall zum Absaugen von Flüssigkeiten, Geweberesten und dergleichen durch den Flußkanal 28 gemäß den Pfeilen 41, 42 und 44 ist die Kugel 36 aus der in Fig. 2 dargestellten Schließstellung im wesentlichen in Richtung quer zur Längsrichtung des Flußkanals 28, d.h. in Richtung eines Pfeiles 46, in eine Freigabestellung gemäß Fig. 3 bewegbar.

Diese Bewegung der Kugel 36 von der Schließstellung in die Freigabestellung wird mittels des Betätigungsorgans 35 aktiv bewerkstelligt, ebenso die umgekehrte Bewegung der Kugel 36 von der Freigabestellung in Fig. 3 in die Schließstellung in Fig. 2. Das Betätigungsorgan 35 ist dazu in Richtung quer zum Flußkanal 28 bewegbar.

Das Betätigungsorgan 35 ist in Form eines Schiebers ausgebildet, der eine Aufnahme 50 für die Kugel 36 aufweist.

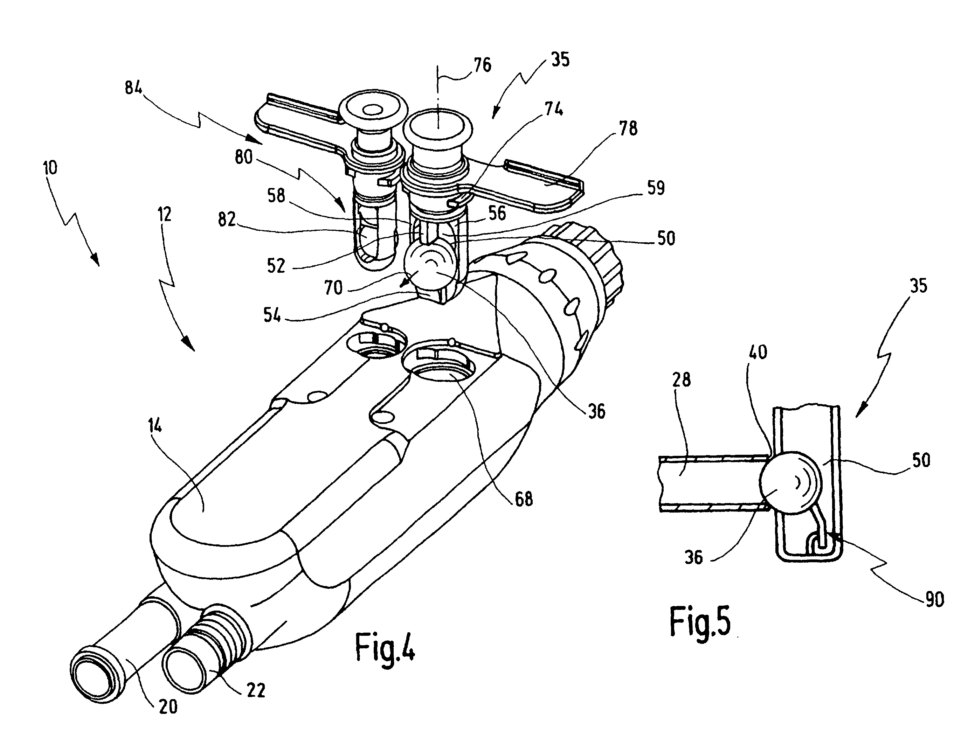

Die Aufnahme 50 ist gemäß Fig. 2 und 4 in der Art eines Käfigs durch Halteelemente 52 und 54, die die Kugel 36 in Betätigungsrichtung vorn und hinten bzw. in Fig. 2 bis 4 oben und unten einfassen, und durch zur Betätigungsrichtung seitliche Halteelemente 56 und 58 gebildet, die die Kugel im wesentlichen seitlich einfassen. Auf diese Weise ist die Kugel 36 in der Aufnahme 50 in Richtungen, die quer zum Flußkanal 28 gerichtet sind, unbeweglich gehalten. Im Bereich des Halteelements 52 lassen die seitlichen Halteelemente 56 und 58 und das Halteelement 52 eine Öffnung 59 für den Durchtritt des Fluids frei.

Die Kugel 36 besitzt in der Aufnahme 50 des Betätigungsorgans 35 in Längsrichtung des Flußkanals 28 eine Beweglichkeit, die zum distalen Ende 38 des proximalen Abschnitts 30 des Flußkanals 28 durch den Dichtrand 40 begrenzt ist und in entgegengesetzter Richtung durch einen Anschlag 60.

Darüber hinaus ist die Kugel 36 in der Aufnahme 50 drehbar gelagert, und zwar im wesentlichen um ihren Mittelpunkt.

Durch die Beweglichkeit der Kugel 36 in Längsrichtung des Flußkanals 28 bewegt sich die Kugel 36 bei der Bewegung von der Schließstellung gemäß Fig. 2 in die Freigabestellung gemäß Fig. 3 und umgekehrt leicht um den unteren Bereich des Dichtrands 40 des Flußkanals 28 herum, ohne daß hohe Bedienkräfte aufgewandt werden müssen. Aufgrund der drehbaren Lagerung der Kugel 36 in der Aufnahme 50 des Betätigungsorgans 35 rollt die Kugel 36 zusätzlich auf dem in Betätigungsrichtung vorderen bzw. in Fig. 2 und 3 unteren Bereich des Dichtrands 40 ab, wodurch die Reibungskräfte beim Bewegen der Kugel 36 aus der Schließstellung in die Freigabestellung und umgekehrt noch weiter verringert sind.

Die Kugel 36 ist des weiteren formelastisch, so daß sie sich in der Schließstellung gemäß Fig. 2 mit besonders hoher Dichtwirkung an den Dichtrand 40 des Flußkanals 28 anschmiegt.

Das Betätigungsorgan 35 weist ferner einen Druckknopf 62 auf, der über einen Stößel 63 mit der Aufnahme 50 zum Bewegen der Kugel 36 verbunden ist. Der Druckknopf 62 läßt sich bequem mit dem Daumen der Hand bedienen, die den Haltegriff 14 hält.

Das Betätigungsorgan 35 ist mittels einer Feder 64 in die Schließstellung der Kugel 36 vorgespannt. Die Feder 64 stützt sich zwischen dem Druckknopf 62 und einem gegenüber dem Instrumentenkörper 12 unbeweglichen Befestigungsteil 66 des Betätigungsorgans 48 ab, wobei der Druckknopf 62 zusammen mit dem Stößel 63 relativ zu dem Befestigungsteil 66 beweglich ist.

In der in Fig. 2 dargestellten Schließstellung der Kugel 36 ist der an dem Flußkanal 28 anstehende Saugstrom unterbrochen. Wenn mit dem Instrument 10 nun abgesaugt werden soll, wird der Druckknopf 62 gemäß Fig. 3 heruntergedrückt, wodurch die Kugel 36 unter Abrollen oder überlagertes Abrollen und Gleiten am in Betätigungsrichtung vorderen bzw. in Fig. 2 und 3 unteren Bereich des Dichtrands 40, wobei die Kugel 36 dabei sich geringfügig auch axial nach distal bewegt, in die Freigabestellung in Fig. 3 bewegt wird.

Das Betätigungsorgan 35 mit der Kugel 36 ist in dem Instrumentenkörper 12, d.h. im vorliegenden Fall im Handgriff 14 in einer Öffnung 68 in Form einer Sacklochbohrung im Handgriff 14 aufgenommen. Das Betätigungsorgan 35 ist dabei zusammen mit der Kugel 36 vom Instrumentenkörper 12, d.h. im vorliegenden Fall von dem Handgriff 14, abnehmbar, wie in Fig. 4 dargestellt ist.

In dem in Fig. 4 dargestellten, vom Instrumentenkörper 12 abgenommenen Zustand läßt sich die Kugel 36 ferner vom Betätigungsorgan 35 abnehmen, beispielsweise im Fall, daß die Kugel 36 aufgrund von Verschleiß ausgetauscht werden soll. Dazu läßt sich die Kugel 36 aus den Halteelementen 52, 54, 56 und 58 in Richtung eines Pfeiles 70 herausnehmen.

Zum Verriegeln bzw. Entriegeln des Betätigungsorgans 35 am Instrumentenkörper 12 ist ein Verriegelungsmechanismus in Form eines Bajonettverschlusses vorgesehen, der eine Nut 72 am Instrumentenkörper 12 und einen entsprechenden Vorsprung 74 am Betätigungsorgan 35 umfaßt. Zum Verriegeln bzw. Entriegeln ist des weiteren ein um eine Längsachse 76 verschwenkbarer Hebel 78 vorgesehen, der in der Verriegelungsstellung gemäß Fig. 1 etwa in Längsrichtung des Instruments 10 angeordnet ist und in der Entriegelungsstellung gegenüber dieser Stellung um etwa 90° gemäß Fig. 4 verschwenkt ist.

Wie bereits erwähnt, weist das Instrument 10 neben dem als Saugkanal ausgebildeten Flußkanal 28 einen dazu parallel verlaufenden zweiten Flußkanal auf, der als Spülkanal ausgebildet ist. Diesem Spülkanal ist in entsprechender Ausgestaltung ein Absperrorgan 80 in Form einer Kugel 82 (Fig. 4) und ein Betätigungsorgan 84 zugeordnet. Die Ausgestaltung des Betätigungsorgans 84 in Verbindung mit der Kugel 82 entspricht dabei der Ausgestaltung des Betätigungsorgans 48 in Verbindung mit der Kugel 36. Da jedoch die Flußrichtung in dem Spülkanal der Flußrichtung in dem Saugkanal entgegengesetzt ist, ist die Kugel 82 an einem nicht näher dargestellten proximalen Ende eines nicht näher dargestellten distalen Abschnitts des Spülkanals angeordnet, an dem wiederum ein Dichtrand vorhanden ist, an dem die Kugel 82 in der Schließstellung dichtend anliegt, und zwar aufgrund des in dem Spülkanal von proximal nach distal gerichteten Spüldruckes.

In Fig. 5 ist ein gegenüber dem zuvor beschriebenen Ausführungsbeispiel geringfügig abgewandeltes Ausführungsbeispiel in einer Prinzipskizze dargestellt, bei dem die Kugel 36 in der Aufnahme 50 des Betätigungsorgans 35 nicht frei drehbar gelagert ist, sondern über eine Art Lasche oder Hebel 90 am Betätigungsorgan befestigt ist. Es versteht sich, daß die Befestigung lösbar ist, um die Kugel 36 vom Betätigungsorgan abnehmen zu können.

Der Hebel oder die Lasche 90 verleihen der Kugel 36 eine Vorspannung in Richtung zum Dichtrand 40 des Flußkanals 28, wodurch die Kugel 36 an diesen in der Schließstellung angedrückt wird. Auf diese Weise gewährleistet die Vorspannung auch dann eine ausreichende Dichtwirkung der Kugel 36, wenn der Strömungsdruck, das Druckgefälle oder der Druck - Volumenstrom - Quotient im Flußkanal 28 gering ist.

## Patentansprüche

1. Medizinisches Instrument zum Spülen und/oder Saugen, mit einem Instrumentenkörper (12), in dem zumindest ein Flußkanal (28) für den Durchtritt eines Fluids vorhanden ist, mit zumindest einem Absperrorgan (34, 80) zum wahlweisen Absperren des zumindest einen Flußkanals (28), und mit einem Betätigungsorgan (35, 84) zum Betätigen des Absperrorgans (34, 80), wobei das zumindest eine Absperrorgan (34, 80) eine Kugel (36, 82) ist, die in Schließstellung an einem Dichtrand (40) des Flußkanals (28) dichtend anliegt, so daß sie den Innenquerschnitt des Flußkanals (28) verschließt, und wobei das Betätigungsorgan (35, 84) einen Druckknopf (62) zur Bedienung des Betätigungsorgans (35, 84) aufweist, **dadurch gekennzeichnet, daß** die Kugel (36, 82) formelastisch ist, daß die Kugel (36, 82) zwischen der Schließstellung zum Verschließen des Flußkanals (28) und einer Freigabestellung zum Freigeben des Flußkanals (28) im wesentlichen in Richtung quer zur Längsrichtung des Flußkanals (28) bewegbar ist, daß das Betätigungsorgan (35, 84) zum Bewegen der Kugel (36, 82) zwischen der Schließstellung und der Freigabestellung ebenfalls im wesentlichen quer zur Längsrichtung des Flußkanals (28) bewegbar ist und daß das Betätigungsorgan (35, 84) zusammen mit der Kugel (36, 82) vom Instrumentenkörper (12) abnehmbar ist.

2. Instrument nach Anspruch 1, **dadurch gekennzeichnet, daß** die Kugel (36, 82) stromaufwärts des Dichtrands (40) des Flußkanals (28) angeordnet ist.

3. Instrument nach Anspruch 1 oder 2, **dadurch gekennzeichnet, daß** die Kugel (36, 82) in einer Aufnahme (50) des Betätigungsorgans (35, 84) angeordnet ist, in der die Kugel (36, 82) eine Beweglichkeit in Längsrichtung des Flußkanals (28) aufweist.

4. Instrument nach Anspruch 3, **dadurch gekennzeichnet, daß** die Kugel (36, 82) um im wesentlichen ihren Mittelpunkt drehbar in der Aufnahme (50) gelagert ist.

5. Instrument nach Anspruch 3 oder 4, **dadurch gekennzeichnet, daß** die Beweglichkeit der Kugel (36, 82) in der Aufnahme (50) entgegen der Flußrichtung des Fluids begrenzt ist.

6. Instrument nach einem der Ansprüche 3 bis 5, **dadurch gekennzeichnet, daß** die Kugel (36, 82) in der Aufnahme (50) in Richtung quer zum Flußkanal (28) im wesentlichen unbeweglich gehalten ist.

7. Instrument nach einem der Ansprüche 1 bis 6, **dadurch gekennzeichnet, daß** das Betätigungsorgan (35, 84) in die Schließstellung oder die Freigabestellung der Kugel (36, 82) vorgespannt ist.

8. Instrument nach einem der Ansprüche 1 bis 7, **dadurch gekennzeichnet, daß** am Betätigungsorgan (35, 84) und am Instrumentenkörper (12) ein Verriegelungsmechanismus zum Verriegeln des Betätigungsorgans (35, 84) am Instrumentenkörper (12) in Form eines Bajonettverschlusses vorhanden ist.

9. Instrument nach einem der Ansprüche 1 bis 8, **dadurch gekennzeichnet, daß** zum Verriegeln und zum Entriegeln des Verriegelungsmechanismus ein um eine Längsachse (76) des Betätigungsorgans (35, 84) verschwenkbarer Hebel (78) vorgesehen ist.

10. Instrument nach einem der Ansprüche 1 bis 9, **dadurch gekennzeichnet, daß** in dem Instrumentenkörper (12) zumindest zwei Flußkanäle (28) vorhanden sind, wobei jedem Flußkanal (28) ein Absperrorgan (34, 80) und ein Betätigungsorgan (35, 84) zugeordnet ist.

11. Instrument nach einem der Ansprüche 1 bis 10, ausgenommen Anspruch 4, **dadurch gekennzeichnet, daß** die Kugel (36, 82) in Richtung zum Dichtrand (40) des Flußkanals (28) hin vorgespannt ist.

12. Instrument nach einem der Ansprüche 1 bis 11, **dadurch gekennzeichnet, daß** der Druckknopf (62) über einen Stößel (63) mit der Aufnahme (50) verbunden und das Betätigungsorgan (35, 84) mittels einer Feder (64) in die Schließstellung vorgespannt ist, die sich zwischen dem Druckknopf (62) und einem gegenüber dem Instrumentenkörper (12) unbeweglichen Befestigungsteil (66) abstützt, wobei der Druckknopf (62) zusammen mit dem Stößel (63) relativ zu dem Befestigungsteil (66) beweglich ist.

## Claims

1. A medical instrument for irrigation and/or suction, comprising an instrument body (12) in which at least one flow channel (28) is present for the passage of a fluid, at least one shut-off member (34, 80) for selectively shutting off the at least one flow channel (28), and an actuating member (35, 84) for actuating the shut-off member (34, 80), wherein the at least one shut-off member (34, 80) is a ball (36, 82) which, in a closure position, lies sealingly on a sealing edge (40) of the flow channel (28) so that it closes off the internal cross section of the flow channel (28), and wherein the actuating member (35, 84) comprises a press-button (64) for operating the actuating member (35, 84), **characterized in that** the ball (36, 82) is elastic in shape, that the ball (36, 82) is movable, between the closure position for closing the flow channel (28) and a release position for freeing the flow channel (28), substantially in a direction transverse to the longitudinal direction of the flow channel (28), that the actuating member (35, 84) for moving the ball (36, 82) between the closure position and the release position is likewise movable substantially in a direction transverse to the longitudinal direction of the flow channel (28), and that the actuating member (35, 84) can be removed from the instrument body (12) together with the ball (36, 82).

2. The instrument of claim 1, **characterized in that** the ball (36, 82) is arranged upstream of the sealing edge (40) of the flow channel (28).

3. The instrument of claim 1 or 2, **characterized in that** the ball (36, 82) is arranged in a receiving seat (50) of the actuating member (35, 84), in which receiving seat (50) the ball (36, 82) has a mobility in the longitudinal direction of the flow channel (28).

4. The instrument of claim 3, **characterized in that** the ball (36, 82) is mounted so as to be rotatable substantially about its midpoint in the receiving seat (50).

5. The instrument of claim 3 or 4, **characterized in that** the mobility of the ball (36, 82) in the receiving seat (50) counter to the direction of flow of the fluid is limited.

6. The instrument of anyone of claims 3 through 5, **characterized in that** the ball (36, 82) in the receiving seat (50) is held substantially immovably in the direction transverse to the flow channel (28).

7. The instrument of anyone of claims 1 through 6, **characterized in that** the actuating member (35, 84) is prestressed into the closure position or the release position of the ball (36, 82).

8. The instrument of anyone of claims 1 through 7, **characterized in that** a locking mechanism in the form of a bayonet catch is present on the actuating member (35, 84) and on the instrument body (12) for the purpose of locking the actuating member (35, 84) on the instrument body (12).

9. The instrument of anyone of claims 1 through 8, **characterized in that**, in order to lock and unlock the locking mechanism, a lever (78) is provided which can be pivoted about a longitudinal axis (76) of the actuating member (35, 84).

10. The instrument of anyone of claims 1 through 9, **characterized in that** at least two flow channels (28) are present in the instrument body (12), and each flow channel (28) is assigned a shut-off member (34, 80) and an actuating member (35, 84).

11. The instrument of anyone of claims 1 through 10, excluding claim 4, **characterized in that** the ball (36, 82) is prestressed in the direction toward the sealing edge (40) of the flow channel (28).

12. The instrument of anyone of claims 1 through 11, **characterized in that** the press-button (62) is connected to the receiving seat (50) via a ram (63), and the actuating member (35, 84) is pre-stressed by means of a spring (64) into the closure position, the spring being supported between the press-button (62) and a securing part (66), wherein the press-button (62) is movable together with the ram (63) relative to the securing part (66).

## Revendications

1. Instrument médical pour laver et/ou aspirer, comportant un corps d'instrument (12) dans lequel se trouve au moins un canal d'écoulement (28) pour le passage d'un fluide, comportant au moins un organe d'arrêt (34, 80) pour fermer au choix le ou les canaux d'écoulement (28), et comportant un organe d'actionnement (35, 84) pour actionner l'organe d'arrêt (34, 80), le ou les organes d'arrêt (34, 80) étant une bille (36, 82) qui, en position de fermeture, s'applique de manière étanche contre un bord d'étanchéité (40) du canal d'écoulement (28), de manière à obturer la section transversale intérieure du canal d'écoulement (28), et l'organe d'actionnement (35, 84) comportant un bouton-poussoir (62) pour commander l'organe d'actionnement (35, 84), **caractérisé en ce que** la bille (36, 82) est de forme élastique, **en ce que** la bille (36, 82) est déplaçable entre la position de fermeture pour obturer le canal d'écoulement (28) et une position de dégagement pour dégager le canal d'écoulement (28) essentiellement dans une direction transversale à la direction longitudinale du canal d'écoulement (28), **en ce que** l'organe d'actionnement (35, 84) destiné à déplacer la bille (36, 82) est déplaçable entre la position de fermeture et la position de dégagement également sensiblement transversalement à la direction longitudinale du canal d'écoulement (28), et **en ce que** l'organe d'actionnement (35, 84) peut être enlevé avec la bille (35, 82) du corps d'instrument (12).

2. Instrument selon la revendication 1, **caractérisé en ce que** la bille (36, 82) est disposée en amont du bord d'étanchéité (40) du canal d'écoulement (28).

3. Instrument selon la revendication 1 ou 2, **caractérisé en ce que** la bille (36, 82) est disposée dans un logement (50) de l'organe d'actionnement (35, 84) dans lequel la bille (36, 82) présente une mobilité dans la direction longitudinale du canal d'écoulement (28).

4. Instrument selon la revendication 3, **caractérisé en ce que** la bille (36, 82) est montée dans le logement (50) de manière à pouvoir tourner sensiblement autour de son centre.

5. Instrument selon la revendication 3 ou 4, **caractérisé en ce que** la mobilité de la bille (36, 82) dans le logement (50) est limitée dans le sens contraire au sens d'écoulement du fluide.

6. Instrument selon l'une des revendications 3 à 5, **caractérisé en ce que** la bille (36, 82) est maintenue sensiblement immobile dans le logement (50) dans la direction transversale au canal d'écoulement (28).

7. Instrument selon l'une des revendications 1 à 6, **caractérisé en ce que** l'organe d'actionnement (35, 84) est précontraint dans la position de fermeture ou la position de dégagement de la bille (36, 82).

8. Instrument selon l'une des revendications 1 à 7, **caractérisé en ce que** sur l'organe d'actionnement (35, 84) du corps d'instrument (12) est prévu un mécanisme de verrouillage pour verrouiller l'organe d'actionnement (35, 84) sur le corps d'instrument (12), sous la forme d'une fermeture à baïonnette.

9. Instrument selon l'une des revendications 1 à 8, **caractérisé en ce que** pour verrouiller et déverrouiller le mécanisme de verrouillage il est prévu un levier (78) pouvant pivoter autour d'un axe longitudinal (76) de l'organe d'actionnement (35, 84).

10. Instrument selon l'une des revendications 1 à 9, **caractérisé en ce que** dans le corps d'instrument (12) sont prévus au moins deux canaux d'écoulement (28), à chaque canal d'écoulement (28) étant associé un organe d'arrêt (34, 80) et un organe d'actionnement (35, 84).

11. Instrument selon l'une des revendications 1 à 10, à l'exception de la revendication 4, **caractérisé en ce que** la bille (36, 82) est précontrainte en direction du bord d'étanchéité (40) du canal d'écoulement (28).

12. Instrument selon l'une des revendications 1 à 11, **caractérisé en ce que** le bouton-poussoir (62) est relié au logement par un poussoir (63), et l'organe d'actionnement (35, 84) est précontraint dans la position de fermeture au moyen d'un ressort (46) qui prend appui entre le bouton-poussoir (62) et un élément de fixation (66) immobile par rapport au corps d'instrument (12), le bouton-poussoir (62) étant déplaçable avec le poussoir (63) par rapport à l'élément de fixation (66).
